# EUROPEAN PATENT SPECIFICATION

(11) **EP 0 664 127 B1**
(45) Date of publication and mention of the grant of the patent: **06.03.2002**
(21) Application number: 94120240.0
(22) Date of filing: 20.12.1994
(51) Int. Cl.: A61K 31/472

(54) **Pharmaceutical compositions containing isoquinoline derivatives**
Pharmazeutische Zusammensetzungen die Isochinolinderivate enthalten
Composition pharmaceutiques contenant des dérivés de l'isoquinoline

(30) Priority: 28.12.1993 JP 35148193
(43) Date of publication of application: 26.07.1995
(73) Proprietor: NIPPON ZOKI PHARMACEUTICAL CO., LTD., Chuo-ku, Osaka (JP)
(72) Inventor: Imai, Shoichi, Miura-gun, Kanagawa (JP); Mabuchi, Ken-ichi, c/o Nippon Zoki, Chuo-ku, Osaka (JP); Kawamura, Minoru, c/o Inst. of Bio-Active-Science, Yashiro-cho, Katoh-gun, Hyogo (JP)
(74) Representative: Hansen, Bernd, Dr. Dipl.-Chem.

(56) References cited:
- EP-A- 0 227 356
- EP-A- 0 513 997
- WO-A-92/03137
- GB-A- 2 181 432
- US-A- 4 956 371
- MATTYUS, A.: "Comparison of No-Spa and Vasalgin for the treatment of headaches associated with vasomotor and cerebral disorders" THER. HUNG. (1968), 16(2), 71-4 , XP002117606
- DATABASE CHEMABS [Online] CHEMICAL ABSTRACTS SERVICE, COLUMBUS, OHIO, US KAPUI, ZOLTAN ET AL: "Indomethacin salts." retrieved from STN Database accession no. 120:62267 XP002117610 & HU 63 387 A (CHINOIN GYOGYSZER ES VEGYESZETI TERMEKEK GYARA RT., HUNG.) 1991
- STOICHEV, TS. ET AL: "Screening pharmacological studies of four dihydroisoquinoline derivatives" ACTA PHYSIOL. PHARMACOL. BULG. ( 1979 ), 5(3), 82-90 , XP002117607
- LUGNIER, C. ET AL: "Analysis of the specificity of inhibitors against some cyclic phosphodiesterases by multiparametric techniques" PHARMAZIE ( 1992 ), 47(1), 46-9 , XP002050965
- KUKOVETZ, WALTHER R. ET AL: "Inhibition of cyclic-3',5'-nucleotide- phosphodiesterase as a possible mode of action of papaverine and similarly acting drugs" NAUNYN-SCHMIEDEBERGS ARCH. PHARMAKOL. ( 1970 ), 267(2), 189-94 , XP002117608
- POECH, GERALD ET AL: "Differentiation of intestinal smooth muscle relaxation caused by drugs that inhibit phosphodiesterase" NAUNYN-SCHMIEDEBERG'S ARCH. PHARMACOL. ( 1976 ), 293(3), 257-68 , XP002117609

## Description

The present invention relates to the use of isoquinoline derivatives and/or pharmaceutically-acceptable salts thereof for the preparation of a pharmaceutical composition

An action of regulating the cell functions of CAMP and cGMP as intracellular information transmitters has been clarified and investigations have also been made into the cyclic nucleotide phosphodiesterases which are enzymes that decompose the above substances. The cyclic nucleotide phosphodiesterases are often abbreviated as phosphodiesterases. They are classified into several isozymes and the molecular structure, the regulating functions, the distributions in tissues and in cells and the specificity of each of these isozymes have been clarified. At the same time, there have been brief investigations into inhibitors which are specific to the isozymes of the phosphodiesterases and some inhibitors which are useful as pharmaceuticals have been found.

Phosphodiesterase type IV isozyme (hereinafter, referred to as PDEIV) which is one of the isozymes of the phosphodiesterases is distributed in tissues and cells such as brain, kidney, spermary, inflammatory cells, etc. It has a low Km value to cAMP and is an enzyme which specifically decomposes cAMP. When the PDEIV is inhibited, the concentration of cAMP in the tissues increases and, therefore, it has been known that the inhibitors for PDEIV exhibit various pharmacological actions such as an action to central nerves such as antidepressant, tranquillizing and antidemential actions, an antiiflammatory action due to inhibition of isolation of chemical mediators and also to inhibition of perfusion of neutrophils, a bronchodilating action, a relaxing action to smooth muscles, a protective action to livers, a diuretic action, etc.

With respect to isoquinoline derivatives, some of them have been known to exhibit pharmacological actions such as a pheripheral dilating action and an antispasmodic action. In fact, drotaverine and the like have already been used as pharmaceuticals. The present inventors carried out an extensive study on the new pharmacological actions of isoquinoline derivatives and found that the isoquinoline derivatives of the present invention have an excellent inhibiting activity with a high specificity to PDEIV.

An object of the present invention is to offer novel pharmaceutical compositions containing isoquinoline derivatives having an excellent PDEIV-inhibiting action as an active component.

The active component of the pharmaceutical composition disclosed in the present specification is an isoquinoline derivative represented by the following general formula: 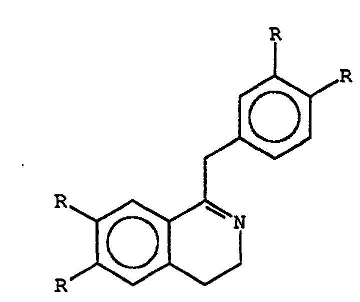 [in which R is an ethoxy group]

In the above formula, R is ethoxy. The isoquinoline derivatives according to the present invention also include pharmaceutically acceptable salts of the compounds repesented by the above general formula such as addition salts with hydrochloric acid, sulfuric acid, nitric acid, hydrobromic acid, phosphoric acid, perchloric acid, thiocyanic acid, boric acid, formic acid, acetic acid, haloacetic acid, propionic acid, glycolic acid, citric acid, tartaric acid, succinic acid, gluconic acid, lactic acid, malonic acid, fumaric acid, anthranilic acid, benzoic acid, cinnamic acid, p-toluenesulfonic acid, naphthalenesulfonic acid, sulfanilic acid, theophylline-acetic acid, etc. Amongst these salts with hydrochloric acid and theophylline-acetic acid are preferred.

These salts may be manufactured from the isoquinoline derivatives according to the present invention in a free state or mutually converted from one to another by conventional means. Stereoisomers such as cis-trans isomers, optical isomers, conformational isomers, etc. or hydrates of these compounds are also included in the present invention.

Examples of compounds according to the present invention which are particularly preferred are as follows:
1-(3',4'-Diethoxybenzyl)-6,7-diethoxy-3,4-dihydroisoquinoline [Compound 3]
1-(3',4'-Diethoxybenzyl)-6,7-diethoxy-3,4-dihydroisoquinoline hydrochloride [Compound 4]
1-(3',4'-Diethoxybenzyl)-6,7-diethoxy-3,4-dihydroisoquinolinetheophylline-7-acetate [Compound 5]
1-(3',4'-Diethoxybenzyl)-6,7-diethoxy-3,4-dihydroisoquinolinetheophylline-7-acetate monohydrate [Compound 6]

The above compounds have been disclosed, for example, in the Japanese Laid-Open Patent Publications 50/046818, 62/167781 and 62/187411. They can be prepared by known methods.

In the following pharmacological actions of the isoquinoline derivatives of the present invention will be shown.

### 1. Preparation of Phosphodiesterase Isozyme.

Each of the isozymes of the phosphodiesterases was prepared by conventional means. Thus, 10 g of freeze-dried powder of porcine artery was homogenized by adding 5 times as much (v/w) of a buffer (comprising 0.25 M of sucrose, 10 mM of Tris hydrochloride (pH 7.8), 5 mM magnesium chloride, 0.2 mM ethylenebis(hydroxyethylenenitrilo)tetraacetic acid, 100 nM p-toluenesulfonyl fluoride, 100 nM leupeptine and 100 nM pepstatin) thereto. The mixture was centrifuged at 2,500 rpm for 20 minutes and the supernatant liquid was further centrifuged at 40,000 rpm for one hour. The resulting supernatant liquid was filtered and dialyzed to a solution (70 mM sodium acetate of pH 6.8 and 5mM of 2-mercaptoethanol) containing the same protease inhibitor as in the above buffer.

The sample obtained by the dialysis was added to a DEAE-sepharose-FF column (Pharmacia) which was filled in a column equilibrated with the above-mentioned buffer and then eluted with a solution of sodium acetate having a linear graded concentration gradient of 0.07-1.0 M. The activity of the phosphodiesterase on the isozyme in the eluted fractions was measured. Fractions in which the activity was concentrated were collected for use as an enzyme source. In the present test system, isozymes of the types I, II, III, IV and V were eluted/fractionated into the fractions of 20 and 21; 30 and 31; 29; 61; and 67, respectively.

### 2. Method of Measuring the Phosphodiesterase Activity.

A reaction system was prepared containing 50 mM Tris HCI (pH: 7.5), 6 mM magnesium chloride, 1 mM of ethylenebis-(hydroxyethylenenitrilo)tetraacetic acid, 2.5 mM dithiothreitol, 2.5% dimethyl sulfoxide, 0.025 mg/ml 5'-nucleotide, 0.23 mg/ml bovine serum albumin and 1-5 µg of each enzyme (the figures represent the final concentrations). The reaction mixture solution was pre-incubated at 30°C for 5-10 minutes, [³H]cAMP or [³H]cGMP was added thereto and the mixture was incubated at 30°C for 10-15 minutes. A cationic exchange resin suspended in water was added to the reaction solution in order to absorb the unreacted cAMP or cGMP and thus stop the reaction. The mixture was then centrifuged at 2,000 g for five minutes, a part of the supernatant liquid was taken out and subjected to a measurement of its radioactivity by means of a liquid scintillation counter. The substance to be tested dissolved in dimethyl sulfoxide or in water was added to the above reaction system and the inhibitory activity of said substance to each isozyme of the phosphodiesterase was measured.

When the inhibitory activity of the test substance against the isozyme of type I was measured, [³H]cAMP was used as a substrate and 5 µg/ml calmodulin was made coexistent. In the case of the inhibitory activity measurement to the type II, [³H]cAMP was used as a substrate and 10 µM cGMP was made coexistent. In the case of the type III, [³H]cAMP was used as a substrate and 100 µM Ro20-1724 (4-[(3-butoxy-4-methoxyphenyl)methyl]-2-imidazolidinone; an inhibitor which is specific to the type IV) was made coexistent. In the case of the type IV, [³H]cAMP was used as a substrate and 10 µM of cilostazol (an inhibitor which is specific to the type III) was made coexistent. In the case of the measurement of the type V, [³H]cGMP was used as a substrate.

The inhibitory activity to phosphodiesterase isozymes was measured according to the above-mentioned test method and the results show that the compounds according to the present invention exhibited a strong inhibitory action especially to the isozyme of the type IV. An example of the results is given in Table 1.

**Table 1**

| Substances Tested | Inhibitory Activity (IC50) |
|---|---|
| Compound 4 of this Invention | 8.1 µM |
| Compound 6 of this Invention | 17.2 µM |
| Ro20-1724 (Control for Comparison) | 26.1 µM |

It is apparent from the result given in Table 1 that the compounds according to the present invention exhibited a PDEIV-inhibiting activity the same as or even higher than that of Ro20-1724 which was known as a very specific RDEIV inhibitor. Furthermore, it can be understood that the resulting PDEIV-inhibiting activity was very specific. In fact, it was at least 10 times stronger than that of of other types of PDE isozymes.

As mentioned already, the concentration of cAMP in the tissue increases when the PDEIV is inhibited and therefore actions on central nerves such as antidepressive action, tranquilizing action, antidemential action, etc., anti-inflammatory action due to inhibiting the isolation of chemical mediators and the perfusion of neutrophils, bronchodilating action, relaxing action for smooth muscles, liver-protecting action, diuretic action, etc. have been suggested as the pharmcological effect of the PDEIV inhibitors.

Consequently, the compounds according to the present invention are of great use in pharmaceuticals such as anti-depressive agents, tranquilizing agents, antidemential agents, antiinflammatory agents, antiallergic agents, antiasthmatic agents, liver-protecting agents, diuretic agents, etc., and also in medicaments for the prevention and therapy of various diseases including distortions of the central nervous system such as depression and dementia as well as others such as inflammation, allergic diseases, asthma, liver diseases, kidney diseases, etc.

The compounds according to the present invention can be used to formulate pharmaceutical preparations by combining them with suitable pharmaceutical carriers or diluents. The formulations include various types of preparations obtainable by common methods such as solids, semisolids, liquids or aerosol formulations for oral or parenteral administration.

For these preparations, the compounds according to the present invention may be used either on their own or in combination with pharmaceutically-active components.

In the case of preparations for oral administration, the compounds according to the present invention may be used alone or together with commonly used excipients. They may for example be blended with suitable additives (e.g. lactose, sugar, glucose, mannitol, corn starch, potato starch, etc.), binders such as crystalline cellulose, cellulose derivatives, gum arabicum, tragacanth solution, sodium alginate solution, gelatin, etc., disintegrating agents such as corn starch, potato starch, potassium carboxymethylcellulose, etc., lubricating agents such as talc, magnesium stearate, etc. and others including bulking agents, moisturizing agents, buffers, preservatives, perfumes and the like to give tablets, diluted powders, granules or capsules.

Alternatively, suppositories may be prepared by blending with fatty/oily bases (e.g. cacao butter), emulsified bases, watersoluble bases (e.g. Macrogol), hydrophilic bases, etc.

In the case of injections, it is possible to prepare solutions or the suspensions in an aqueous and nonaqueous solvents such as distilled water for injection, physiological saline solution, Ringer's solution, plant oil, synthetic fatty acid glycerides, higher fatty acid esters, propylene glycol, etc.

It is also possible, depending upon the state of the patient and the type of the disease, to prepare pharmaceutical compositions, other than those mentioned above, which are suitable for therapeutic application as, for example, inhalating agents, aerosol agents, ointments, poultices, eye drops, etc.

The preferred dose of the compounds according to the present invention may vary depending upon the patient's individiual requirements (i.e. age, body weight, symptoms, etc.), form of the preparation, method of administration, period of administration etc. In order to achieve the desired effect a 10-2,000 mg per day may normally be administered orally to the average adult either in a single dosage or in several smaller doses.

In the case of a parenteral administration e.g. by injection it is preferable, due to the influence of the absorption, etc., for a quantity of 1/3 to 1/10 of the above oral dose to be administered.

In the following some examples of pharmaceutical formulations containing the compounds according to the present invention as an active component are given.

**Table 2**

| (Formulation Example 1; Tablet) | |
|---|---|
| Components | Amount per Tablet |
| Compound 6 of this Invention | 50 mg |
| Calcium citrate | 118 mg |
| Crystalline cellulose | 10 mg |
| Magnesium stearate | 2 mg |
| | |
| Total | 180 mg |

**Table 3**

| (Formulation Example 2; Injection) | |
|---|---|
| Components | Amount per Ampoule |
| Compound 4 of the Invention | 20 mg |
| Sodium chloride | q.s. |
| Distilled water for injection | q.s. |
| | |
| Total | 1 ml |

## Claims

1. Use of at least one of the isoquinoline derivatives represented by the following general formula 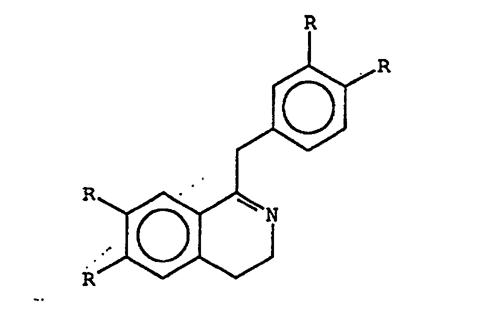 (in which R is an ethoxy group)
and/or a pharmaceutically acceptable salt thereof for the preparations of an anti-depressive agent, a tranquilizer, an anti-allergic agent, an anti-asthmatic agent, a liver-protecting agent or a diuretic agent.

2. The use according to claim 1 wherein the isoquinoline derivative is 1- (3',4'-diethoxybenzyl)-6,7-diethoxy-3,4-dihydroisoquinoline hydrochloride.

3. The use according to claim 1 wherein the isoquinoline derivative is 1-(3',4'-diethoxybenzyl)-6,7-diethoxy-3,4-dihydroisoquinoline-theophylline-7-acetate.

4. The use according to claim 1 wherein the isoquinoline derivative is 1-(3',4'-diethoxybenzyl)-6,7-diethoxy-3,4-dihydroisoquinoline-theophylline-7-acetate monohydrate.

## Patentansprüche

1. Verwendung von mindestens einem Isochinolinderivat, dargestellt durch die folgende allgemeine Formel: 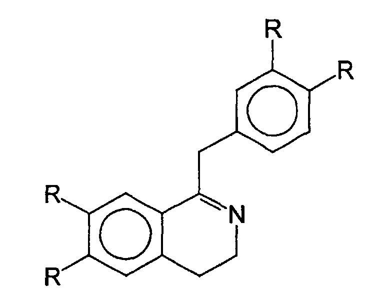 (bei der R eine Ethoxygruppe ist), und/oder eines pharmazeutisch annehmbaren Salzes davon zur Herstellung eines Antidepressivums, eines Beruhigungsmittels, eines Antiallergikums, eines Antiasthmatikums, eines leberschützenden Mittels oder eines Diuretikums.

2. Verwendung gemäss Anspruch 1, wobei das Isochinolinderivat 1-(3',4'-Diethoxybenzyl)-6,7-diethoxy-3,4-dihydroisochinolin-hydrochlorid ist.

3. Verwendung gemäss Anspruch 1, wobei das Isochinolinderivat 1-(3',4'-Diethoxybenzyl)-6,7-diethoxy-3,4-dihydroisochinolin-theophyllin-7-acetat ist.

4. Verwendung gemäss Anspruch 1, wobei das Isochinolinderivat 1-(3',4'-Diethoxybenzyl)-6,7-diethoxy-3,4-dihydroisochinolin-theophyllin-7-acetatmonohydrat ist.

## Revendications

1. Utilisation d'au moins un des dérivés d'isoquinoline représentés par la formule générale suivante 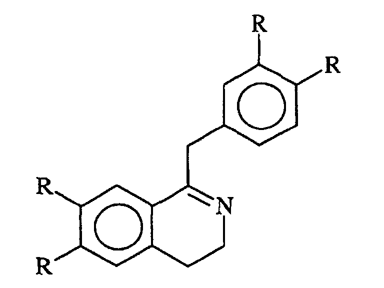 [où R est un groupe éthoxy]
et/ou d'un sel pharmaceutiquement acceptable de ceux-ci pour les préparations d'un agent antidépresseur, d'un tranquillisant, d'un agent antiallergique, d'un agent antiasthmatique, d'un agent de protection du foie ou d'un agent diurétique.

2. Utilisation selon la revendication 1, dans laquelle le dérivé d'isoquinoline est l'hydrochlorure de 1-(3',4'-diéthoxybenzyl)-6,7-diéthoxy-3,4-dihydroisoquinoline.

3. Utilisation selon la revendication 1, dans laquelle le dérivé d'isoquinoline est le 1-(3',4'-diéthoxybenzyl)-6,7-diéthoxy-3,4-dihydroisoquinoline-théophylline-7-acétate.

4. Utilisation selon la revendication 1, dans laquelle le dérivé d'isoquinoline est le monohydrate de 1-(3',4'-diéthoxybenzyl)-6,7-diéthoxy-3,4-dihydroisoquinoline-théophylline-7-acétate.
